# EUROPÄISCHE PATENTSCHRIFT

(11) **EP 2 782 948 B1**
(45) Veröffentlichungstag und Bekanntmachung des Hinweises auf die Patenterteilung: **08.06.2016**
(21) Anmeldenummer: 12787025.1
(22) Anmeldetag: 19.11.2012
(51) Int. Cl.: C08G 73/02

(54) **LIPOPHILE POLYALKYLENPOLYAMINE DURCH HOMOGEN-KATALYSIERTE ALKOHOL-AMINIERUNG**
LIPOPHILIC POLYALKYLENE POLYAMINES OBTAINED BY HOMOGENEOUSLY CATALYZED ALCOHOL AMINATION
POLYALKYLÈNE POLYAMINES LIPOPHILES OBTENUES PAR AMINATION D'ALCOOLS CATALYSÉE PAR CATALYSEUR HOMOGÈNE

(30) Priorität: 25.11.2011 EP 11190791
(43) Veröffentlichungstag der Anmeldung: 01.10.2014
(73) Patentinhaber: BASF SE, 67056 Ludwigshafen am Rhein (DE)
(72) Erfinder: STRAUTMANN, Julia, 68163 Mannheim (DE); SCHAUB, Thomas, 67433 Neustadt (DE); HÜFFER, Stephan, 67063 Ludwigshafen (DE); MAAS, Steffen, 55270 Bubenheim (DE); WOOD, Claudia, 69469 Weinheim (DE)
(74) Vertreter: BASF IP Association
(86) Internationale Anmeldenummer: PCT/EP2012/072944
(87) Internationale Veröffentlichungsnummer: WO 2013/076025

(56) Entgegenhaltungen:
- WO-A1-97/23546
- JP-A- 54 080 400
- US-A- 5 030 740
- US-A- 5 726 284

## Beschreibung

Die vorliegende Erfindung betrifft ein Verfahren zur Herstellung von lipophilen Polyalkylenpolyaminen durch homogen katalytisierte Alkohol-Aminierung von aliphatischen Aminoalkoholen (Alkanolaminen) oder von Di- oder Polyaminen mit Di- oder Polyolen.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils konkret angegebenen Kombination, sondern auch in anderen Kombinationen verwendbar sind, ohne den Rahmen der Erfindung zu verlassen. Bevorzugt bzw. ganz bevorzugt sind die Ausführungsformen der vorliegenden Erfindung in denen alle Merkmale die bevorzugten bzw. ganz bevorzugten Bedeutungen haben.

Polyethylenimine sind wertvolle Produkte mit einer Vielzahl von unterschiedlichen Verwendungen. Beispielsweise werden Polyethylenimine eingesetzt: a) als Haftvermittler für Druckfarben für Laminatfolien; b) als Hilfsmittel (Adhäsion) zur Herstellung von mehrlagigen Komposit-Folien, wobei nicht nur unterschiedliche Polymerschichten, sondern auch Metallfolien kompatibilisiert werden; c) als Haftvermittler für Klebstoffe, beispielsweise in Verbindung mit Polyvinylalkohol, -butyrat, und -acetat und Styrol-Copolymeren, oder als Kohäsionspromotor für Etikettenklebstoffe; d) niedermolekulare Polyethylenimine können zudem als Vernetzer/Härter in Epoxidharzen und Polyurethanklebstoffen verwendet werden; e) als Primer in Lackanwendungen zur Verbesserung der Haftung auf Substraten wie Glas, Holz, Kunststoff und Metall; f) zur Verbesserung der Nasshaftung in Standard-Dispersionsfarben sowie zur Verbesserung der Instantan-Regenbeständigkeit von Anstrichfarben beispielsweise für Fahrbahn-Markierungen; g) als Komplexierungsmittel mit hohem Bindevermögen für Schwermetallen wie Hg, Pb, Cu, Ni und Flockungsmittel in der Wasserbehandlung/Wasseraufbereitung; h) als Penetrationshilfsmittel für aktive Metallsalz-Formulierungen im Holzschutz; i) als Korrosionsinhibitoren für Eisen und Buntmetalle; j) zur Immobilisierung von Proteinen und Enzymen. Für diese Anwendungen lassen sich auch hydrophile, ambiphile und lipophile Polyalkylenpolyamine, welche nicht vom Ethylenimin abgeleitet sind, einsetzen.

Polyethylenimine werden gegenwärtig durch die Homopolymerisation von Ethylenimin erhalten. Ethylenimin ist ein hochreaktives, korrosives und toxisches Zwischenprodukt. (Aziridine, Ulrich Steuerle, Robert Feuerhake; in Ullmann's Encyclopedia of Industrial Chemistry, 2006, Wiley-VCH, Weinheim).

Für die Herstellung von nicht vom Aziridin abgeleiteten Polyalkylenpolyaminen -[(CH₂)ₓN]- mit Alkylengruppen > C₂ (x > 2) gibt es keine der Aziridinroute analoge Verfahren, wodurch es bisher keinen kostengünstigen Prozess zu deren Herstellung gibt.

Die homogen-katalysierte Aminierung von Alkoholen ist aus der Literatur für die Synthese von primären, sekundären und tertiären Aminen ausgehend von Alkoholen und Aminen bekannt, wobei in allen beschriebenen Ausführungen monomere Produkte erhalten werden.

In US 3,708,539 ist die Synthese von primären, sekundären und tertiären Aminen unter Verwendung eines Ruthenium-Phosphan-Komplexes beschrieben.

In Y. Watanabe, Y. Tsuji, Y. Ohsugi Tetrahedron Lett. 1981, 22, 2667-2670 wird über die Herstellung von Arylaminen durch die Aminierung von Alkoholen mit Anilin unter Verwendung von [Ru(PPh₃)₃Cl₂] als Katalysator berichtet.

In EP 0 034 480 A2 wird die Herstellung von N-Alkyl- oder N,N-Dialkylaminen durch die Reaktion von primären oder sekundären Aminen mit einem primären oder sekundären Alkohol unter Verwendung eines Iridium-, Rhodium-, Ruthenium-, Osmium-, Platin-, Palladium- oder Rheniumkatalysators offenbart.

In EP 0 239 934 A1 wird die Synthese von mono- und diaminierten Produkten ausgehend von Diolen wie Ethylenglykol und 1,3-Propandiol mit sekundären Aminen unter Verwendung von Ruthenium- und Iridiumphosphan-Komplexen beschrieben.

In K.I. Fujita, R. Yamaguchi Synlett, 2005, 4, 560-571 wird die Synthese von sekundären Aminen durch die Reaktion von Alkoholen mit primären Aminen sowie die Synthese cyclischer Amine durch die Reaktion von primären Aminen mit Diolen durch Ringschluß unter Verwendung von Iridiumkatalysatoren beschrieben.

In A. Tillack, D. Hollmann, K. Mevius, D. Michalik, S. Bähn, M. Beller Eur. J. Org. Chem. 2008, 4745-4750, in A. Tillack, D. Hollmann, D. Michalik, M. Beller Tetrahedron Lett. 2006, 47, 8881-8885, in D. Hollmann, S. Bähn, A. Tillack, M. Beller Angew. Chem. Int. Ed. 2007, 46, 8291-8294 sowie in M. Haniti, S.A. Hamid, C.L. Allen, G.W. Lamb, A.C. Maxwell, H.C. Maytum, A.J.A. Watson, J.M.J. Williams J. Am. Chem. Soc, 2009, 131, 1766-1774 werden Synthesen von sekundären und tertiären Aminen ausgehend von Alkoholen und primären bzw. sekundären Aminen unter Verwendung von homogenen Rutheniumkatalysatoren beschrieben.

Über die Synthese von primären Aminen durch die Umsetzung von Alkoholen mit Ammoniak unter Verwendung eines homogenen Rutheniumkatalysators wird in C. Gunanathan, D. Milstein Angew. Chem. Int. Ed. 2008, 47, 8661-8664 berichtet.

US 5,726,284 A offenbart ein Verfahren zur Herstellung von vernetzten, in Wasser unlöslichen, polymeren Ammoniumsalzen, welche durch geeignete verknüpfende Gruppen miteinander verbunden sind, es sich bei mindestens 25 % der verknüpfenden Gruppen um C₇- bis C₂₀-Alkylengruppen handelt. Bei den Übrigen verknüpfenden Gruppen kann es sich beispielsweise um Kohlenwasserstoffgruppen mit 2 bis 50 Kohlenstoffatomen handeln, wobei die Gruppen optional mit funktionellen Gruppen substituiert sein können.

JP 54 080400 A offenbart ein Verfahren zur Herstellung von Polyhexamethylenimin durch ringöffnende Polymerisation von Hexamethylenimin in Gegenwart von Metallen der Gruppe VIII bzw. Verbindungen davon bei 50°C bis 300°C.

In unserer älteren Anmeldung WO 2011/151268 A1 werden allgemeine Verfahren zur Herstellung von Polyalkylenpolyaminen durch katalytische Alkohol-Aminierung von Alkanolaminen oder von Di- oder Polyaminen mit Di- oder Polyolen beschrieben.

Aufgabe der vorliegenden Erfindung ist es, ein Verfahren zur Herstellung von lipophilen Polyalkylenpolyaminen zu finden, bei dem keine unerwünschten Koppelprodukte, beispielsweise Ammoniak, gebildet werden.

Diese und andere Aufgaben werden, wie aus dem Offenbarungsgehalt der vorliegenden Erfindung ersichtlich, durch die verschiedenen Ausführungsformen des erfindungsgemäßen Verfahrens zur Herstellung von lipophilen Polyalkylenpolyaminen durch homogen katalysierte Alkohol-Aminierung, bei dem
(i) aliphatische Aminoalkohole miteinander oder
(ii) aliphatische Diamine oder Polyamine mit aliphatischen Diolen oder Polyolen unter Wasserabspaltung in Gegenwart eines homogenen Katalysators umgesetzt werden,
wobei mindestens eines der Edukte aliphatische Aminoalkohole, aliphatische Diamine oder Polyamine oder aliphatische Diole oder Polyole eine Alkyl- oder Alkylengruppe mit fünf oder mehr, bevorzugt sieben oder mehr, besonders bevorzugt neun oder mehr Kohlenstoffatomen enthält und
nach der Umsetzung,
bevorzugt nach Abkühlen, insbesondere auf Raumtemperatur,
bevorzugt nach Zugabe eines, bevorzugt polaren Lösungsmittels, insbesondere Wasser, eine Phasentrennung in mindestens eine unpolare und mindestens eine polare Phase vorliegt, wobei die lipophilen Polyalkylenpolyamine in der unpolaren Phase angereichert vorliegen,
gelöst.

Unter lipophilen Polyalkylenpolyaminen sind fettlösliche Polyamine zu verstehen, die aus Monomeren synthetisiert werden, welche Alkyl- oder Alkylengruppen mit fünf oder mehr Kohlenstoffatomen enthalten.

Unter Raumtemperatur wird 21°C verstanden. Nach der Umsetzung wird das Reaktionsgemisch bevorzugt abgekühlt. Zweckmäßigerweise erfolgt die Abkühlung auf Umgebungstemperatur, insbesondere auf Raumtemperatur. Die Abkühlung unterstützt in der Regel die Phasentrennung in eine unpolare und eine polare Phase.

Unter einem homogenen Katalysator wird ein Katalysator verstanden, der während der Umsetzung homogen gelöst im Reaktionsmedium vorliegt.

Ausdrücke der Form Cₐ-C_{b} bezeichnen im Rahmen dieser Erfindung chemische Verbindungen oder Substituenten mit einer bestimmten Anzahl von Kohlenstoffatomen. Die Anzahl an Kohlenstoffatomen kann aus dem gesamten Bereich von a bis b, einschließlich a und b gewählt werden, a ist mindestens 1 und b immer größer als a. Eine weitere Spezifizierung der chemischen Verbindungen oder der Substituenten erfolgt durch Ausdrücke der Form Cₐ-C_{b}-V. V steht hierbei für eine chemische Verbindungsklasse oder Substituentenklasse, beispielsweise für Alkylverbindungen oder Alkylsubstituenten.

Im Einzelnen haben die für die verschiedenen Substituenten angegebenen Sammelbegriffe folgende Bedeutung:
C₁-C₅₀-Alkyl: geradkettige oder verzweigte Kohlenwasserstoffreste mit bis zu 50 Kohlenstoffatomen, beispielsweise C₁-C₁₀-Alkyl oder C₁₁-C₂₀-Alkyl, bevorzugt C₁-C₁₀-Alkyl beispielsweise C₁-C₃-Alkyl, wie Methyl, Ethyl, Propyl, Isopropyl, oder C4-C6-Alkyl, n-Butyl, sec-Butyl, tert.-Butyl, 1,1-Dimethylethyl, Pentyl, 2-Methylbutyl, 1,1-Dimethylpropyl, 1,2-Dimethylpropyl, 2,2-Dimethylpropyl, 1-Ethylpropyl, Hexyl, 2-Methylpentyl, 3-Methyl-pentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 2-Ethylbutyl, 1,1,2-Trimethylpropyl, 1,2,2-Trimethylpropyl, 1-Ethyl-1-methylpropyl, 1-Ethyl-2-methylpropyl, oder C₇-C₁₀-Alkyl, wie Heptyl, Octyl, 2-Ethyl-hexyl, 2,4,4-Trimethylpentyl, 1,1,3,3-Tetramethylbutyl, Nonyl oder Decyl sowie deren Isomere.

C₃-C₁₅-Cycloalkyl: monocyclische, gesättigte Kohlenwasserstoffgruppen mit 3 bis zu 15 Kohlenstoffringgliedern, bevorzugt C₃-C₈-Cycloalkyl wie Cyclopropyl, Cyclobutyl, Cyclopentyl, Cyclohexyl, Cycloheptyl oder Cyclooctyl sowie ein gesättigtes oder ungesättigtes cyclisches System wie z. B. Norbornyl oder Norbenyl.

Aryl: ein ein- bis dreikerniges aromatisches Ringsystem enthaltend 6 bis 14 Kohlenstoffringglieder, z. B. Phenyl, Naphthyl oder Anthracenyl, bevorzugt ein ein- bis zweikerniges, besonders bevorzugt ein einkerniges aromatisches Ringsystem.

Das Symbol "*" kennzeichnet im Rahmen der vorliegenden Erfindung bei allen chemischen Verbindungen die Valenz über die eine chemische Gruppe an eine andere chemische Gruppe angebunden ist.

Im Folgenden wird die vorliegende Erfindung im Einzelnen erläutert.

Erfindungsgemäß werden die lipophilen Polyalkylenpolyamine -wie bereits beschrieben- durch Umsetzung von (i) aliphatischen Aminoalkoholen miteinander unter Wasserabspaltung oder von (ii) aliphatischen Diaminen oder Polyaminen mit aliphatischen Diolen oder Polyolen unter Wasserabspaltung, jeweils in Gegenwart eines Katalysators, erhalten.

Geeignete aliphatische Aminoalkohole enthalten mindestens eine primäre oder sekundäre Aminogruppe und mindestens eine OH-Gruppe sowie mindestens eine lipophile Alkylen- oder Alkylgruppe mit fünf oder mehr Kohlenstoffatomen. Beispiele sind lineare verzweigte oder cyclische Alkanolamine wie Aminodimethylpentanol, beispielsweise 5-Amino-2,2-dimethylpentanol oder 3-Amino-2,4-dimethyl-2-pentanol, Aminohexanol, beispielsweise 2-Aminohexan-1-ol, Aminoheptanol, beispielsweise 2-Aminoheptan-1-ol, Aminooctanol, beispielsweise 2-Aminooctan-1-ol, Aminononanol, beispielsweise 2-Aminononan-1-ol, Aminodecanol, beispielsweise 2-Aminodecan-1-ol, Aminoundecanol, beispielsweise 2-Aminoundecan-1-ol, Aminododecanol, beispielsweise 2-Aminododecan-1-ol, Aminotridecanol, beispielsweise 2-Aminotridecan-1-ol.

Geeignete aliphatische Diamine enthalten mindestens zwei primäre oder mindestens eine primäre und eine sekundäre oder mindestens zwei sekundäre Aminogruppen, bevorzugt enthalten sie zwei primäre Aminogruppen. Beispiele sind lineare verzweigte oder cyclische aliphatische Diamine. Beispiele sind Ethylendiamin, 1,3-Propylendiamin, 1,2-Propylendiamin, Butylendiamin, beispielsweise 1,4-Butylendiamin oder 1,2-Butylendiamin, Diaminopentan, beispielsweise 1,5-Diaminopentan oder 1,2-Diaminopentan, 1,5-Diamino-2-methylpentan, Diaminohexan, beispielsweise 1,6-Diaminohexan oder 1,2-Diaminohexan, Diaminoheptan, beispielsweise 1,7-Diaminoheptan oder 1,2-Diaminoheptan, Diaminooctan, beispielsweise 1,8-Diaminooctan oder 1,2-Diaminooctan, Diaminononan, beispielsweise 1,9-Diaminononan oder 1,2-Diaminononan, Diaminodecan, beispielsweise 1,10-Diaminodecan oder 1,2-Diaminodecan, Diaminoundecan, beispielsweise 1,11-Diaminoundecan oder 1,2-Diaminoundecan, Diaminododecan, beispielsweise 1,12-Diaminododecan oder 1,2-Diaminododecan, 3,3'-Dimethyl-4,4'-diaminodicyclohexylmethan, 4,4'-Diaminodicyclohexylmethan, Isophorondiamin, 2,2-Dimethylpropan-1,3-diamin, 4,7,10-Trioxatridecan-1,13-diamin, 4,9-Dioxadodecan-1,12-diamin, Polyetheramine, Piperazin, 3-(Cyclohexyl-amino)propylamin, 3-(Methylamino)propylamin, N,N-Bis(3-aminopropyl)methylamin.

Geeignete aliphatische Diole sind lineare verzweigte oder cyclische aliphatische Diole. Beispiele für aliphatische Diole sind Ethylenglykol, 1,2-Propylenglykol, 1,3-Propylenglykol, 2-Methyl-1,3-propandiol, Butandiole, beispielsweise 1,4-Butylenglykol oder Butan-2,3-diol oder 1,2-Butylengylkol, Pentandiole, beispielsweise Neopentylglykol oder 1,5-Pentandiol oder 1,2-Pentandiol, Hexandiole, beispielsweise 1,6-Hexandiol oder 1,2-Hexandiol, Heptandiole, beispielsweise 1,7-Heptandiol oder 1,2-Heptandiol, Octandiole, beispielsweise 1,8-Octandiol oder 1,2-Octandiol, Nonandiole, beispielsweise 1,9-Nonandiol oder 1,2-Nonandiol, Decandiole, beispielsweise 1,10-Decandiol oder 1,2-Decandiol, Undecandiole, beispielsweise 1,11-Undecandiol oder 1,2-Undecandiol, Dodecandiole, beispielsweise 1,12-Dodecandiol, 1,2-Dodecandiol, Tridecandiole, beispielsweise 1,13-Tridecandiol oder 1,2-Tridecandiol, Tetradecandiole, beispielsweise 1,14-Tetradecandiol oder 1,2-Tetradecandiol, Pentadecandiole, beispielsweise 1,15-Pentadecandiol oder 1,2-Pentadecandiol, Hexadecandiole, beispielsweise 1,16-Hexadecandiol oder 1,2-Hexadecandiol, Heptadecandiole, beispielsweise 1,17-Heptadecandiol oder 1,2-Heptadecandiol, Octadecandiole, beispielsweise 1,18-Octadecandiol oder 1,2-Octadecandiol, 3,4-Dimethyl-2,5-hexandiol, PolyTHF, 1,4-Bis-(2-hydroxyethyl)piperazin, Diethanolamine, beispielsweise Butyldiethanolamin oder Methyldiethanolamin.

Erfindungsgemäß werden die lipophilen Polyalkylenpolyamine auch hergestellt aus aliphatischen Diolen mit fünf oder mehr Kohlenstoffatomen und aliphatischen Diaminen oder aus aliphatischen Diolen und aliphatischen Diaminen mit fünf oder mehr Kohlenstoffatomen oder aus aliphatischen Diolen mit fünf oder mehr Kohlenstoffatomen und aliphatischen Diaminen mit fünf oder mehr Kohlenstoffatomen.

In einer bevorzugten Ausführungsform des erfindungsgemäßen Verfahrens enthält mindestens eines der Edukte aliphatische Aminoalkohole, aliphatische Diamine oder Polyamine oder aliphatischen Diole oder Polyole eine Alkyl- oder Alkylengruppe mit von 5 bis 50, bevorzugt von 5 bis 20, besonders bevorzugt von 6 bis 18, ganz besonders bevorzugt von 7 bis 16, insbesondere bevorzugt von 8 bis 14 und insbesondere von 9 bis 12 Kohlenstoffatomen.

Weiterhin bevorzugt enthalten erfindungsgemäß erhältliche Polyalkylenpolyamine C₅-C₅₀-Alkyleneinheiten oder Alkyleinheiten, besonders bevorzugt C₈-C₂₀-Alkyleneinheiten oder Alkyleinheiten. Diese können linear oder verzweigt sein, bevorzugt sind sie linear. Beispiele sind: 1,2-Octylen, 1,2-Nonylen, 1,2-Decylen, 1,2-Undecylen, 1,2-Dodecylen, 1,2-Tridecylen, 1,8-Ocytylen, 1,9-Nonylen, 1,10-Decylen, 1,11-Undecylen, 1,12-Dodecylen, 1,13-Tridecylen, Octyl, Nonyl, Decyl, Undecyl, Dodecyl, Tridecyl.

Es können selbstverständlich auch Gemische von aliphatischen Aminoalkoholen oder Gemische von Alkandiolen oder Gemischen von Diaminoalkanen in den jeweiligen Umsetzungen eingesetzt werden. Die resultierenden Polyalkylenpolyamine können Alkyleneinheiten unterschiedlicher Länge enthalten. Mindestens eines der Edukte enthält dabei eine Alkyl- oder Alkylengruppe mit fünf oder mehr Kohlenstoffatomen.

Es können auch polyfunktionelle Aminoalkohole mit mehr als einer OH-Gruppe oder mehr als einer primären oder sekundären Aminogruppe miteinander umgesetzt werden. Dabei werden hochverzweigte Produkte erhalten. Beispiele für polyfunktionelle Aminoalkohole sind Diethanolamin, N-(2-Aminoethyl)ethanolamin, Diisopropanolamin, Diisononanolamin, Diisodecanolamin, Diisoundecanolamin, Diisododecanolamin, Diisotridecanolamin. Mindestens eines der Edukte enthält dabei eine Alkyl- oder Alkylengruppe mit fünf oder mehr Kohlenstoffatomen.

Es können auch Polyole oder Gemische von Diolen und Polyolen mit Diaminen umgesetzt werden. Es können auch Polyamine oder Gemische von Diaminen und Polyaminen mit Diolen umgesetzt werden. Es können auch Polyole oder Gemische von Diolen und Polyolen mit Polyaminen oder Gemischen von Diaminen und Polyaminen umgesetzt werden. Es können auch Polyaminpolyole oder Gemische von Polyaminpolyolen mit Diolen, Diaminen oder Alkoholaminen umgesetzt werden. Dabei werden hochverzweigte Produkte erhalten. Beispiele für Polyole sind Glycerin, Trimethylolpropan, Sorbitol, Triethanolamin, Triisopropanolamin. Beispiele für Polyamine sind Diethylentriamin, Tris-(aminoethyl)amin, Triazin, 3-(2-Aminoethylamino)propylamin, Dipropylentriamin, N,N'-Bis-(3-aminopropyl)-ethylendiamin. Mindestens eines der Edukte enthält dabei eine Alkyl- oder Alkylengruppe mit fünf oder mehr Kohlenstoffatomen.

In einer Ausführungsform des erfindungsgemäßen Verfahrens werden aliphatische Polyalkylenpolyamine mit aliphatischen Aminoalkoholen oder mit Diaminen oder Polyaminen oder mit aliphatischen Diolen oder Polyolen zu lipohilen Polyalkylenpolyaminen umgesetzt.

Hydroxy- und Aminogruppen in Diolen, Polyolen und Diaminen, Polyamainen werden bevorzugt in Molverhältnissen von 20 : 1 bis 1 : 20 eingesetzt, besonders bevorzugt in Verhältnissen von 8 : 1 bis 1 : 8, insbesondere von 3 : 1 bis 1 : 3.

In einer bevorzugten Ausführungsform des erfindungsgemäßen Verfahrens befinden sich die Heteroatome N oder O eines der Edukte aliphatische Aminoalkohole, aliphatische Diamine oder Polyamine oder aliphatischen Diole oder Polyole in alpha- und beta-Position an der Kette der C-Atome. Alpha - und beta-Position bezeichnet die Positionen an zwei kovalent aneinander gebundenen C-Atomen, wobei eines der C-Atome terminal ist.

In einer bevorzugten Ausführungsform des erfindungsgemäßen Verfahrens befinden sich die Heteroatome N oder O eines der Edukte aliphatische Aminoalkohole, aliphatische Diamine oder Polyamine oder aliphatischen Diole oder Polyole in alpha- und omega-Position an der Kette der C-Atome. Alpha- und omega-Position bedeutet, dass die beiden Heteroatome an terminale C-Atome gebunden sind, die sich an entgegengesetzten Enden der Kette der C-Atome befinden.

Der Katalysator enthält im Allgemeinen mindestens ein Element der Nebengruppen des Periodensystems (Übergangsmetall).

Das erfindungsgemäße Verfahren der Alkohol-Aminierung kann in Gegenwart oder in Abwesenheit eines zusätzlichen Lösungsmittels durchgeführt werden.

Die erfindungsgemäße Alkohol-Aminierung kann in einem mehrphasigen, bevorzugt einphasigen oder zweiphasigen, flüssigen System bei Temperaturen von im Allgemeinen 20 bis 250 °C durchgeführt werden. Nach Beendigung der Umsetzung und gegebenenfalls nach Zugabe eines weiteren Lösungsmittels liegt ein zweiphasiges Reaktionssystem vor. In diesem enthält die eine Phase, häufig die Oberphase, im Wesentlichen den Großteil der gebildeten lipophilen Polyalkylenpolyamine und gegebenenfalls ein unpolares Lösungsmittel sowie noch vorhandene unpolaren Edukte. Die zweite Phase, häufig die Unterphase, enthält im Wesentlichen Wasser und gegebenenfalls ein polares Lösungsmittel sowie bevorzugt den homogen gelösten Katalysator.

In einer bevorzugten Ausführungsform des erfindungsgemäßen Verfahrens wird dem Reaktionsgemisch nach der Umsetzung, insbesondere nach dem Abkühlen auf Raumtemperatur, ein polares Lösungsmittel, beispielsweise Wasser, Dimethylformamid, Dimethylsulfoxid, ionische Flüssigkeiten, Methanol, Ethanol, bevorzugt Wasser, zugesetzt. Das Hinzufügen des polaren Lösungsmittels unterstützt in der Regel die Phasentrennung nach der Umsetzung.

Wie dem Fachmann aus seinem allgemeinen Fachwissen bekannt ist, ist eine Phasentrennung nie vollständig, d.h. geringe Mengen, beispielsweise bis zu 5 % der unpolaren Verbindungen können auch in der zweiten Phase und geringe Mengen, beispielsweise bis zu 5 % der polaren Verbindungen können auch in der ersten Phase gelöst sein.

In einer bevorzugten Ausführungsform der Erfindung werden Alkylendiamine mit 2 oder mehr Kohlenstoffatomen (C-Atomen) in der linearen oder verzweigten Alkylenkette mit Alkandiolen mit fünf oder mehr C-Atomen in der linearen oder verzweigten Alkylenkette oder Alkylendiamine mit fünf oder mehr C-Atomen in der linearen oder verzweigten Alkylenkette mit Alkandiolen mit 2 oder mehr C-Atomen in der linearen oder verzweigten Alkylenkette in Gegenwart eines Katalysators umgesetzt (vgl. beispielhaft Gleichung 1):

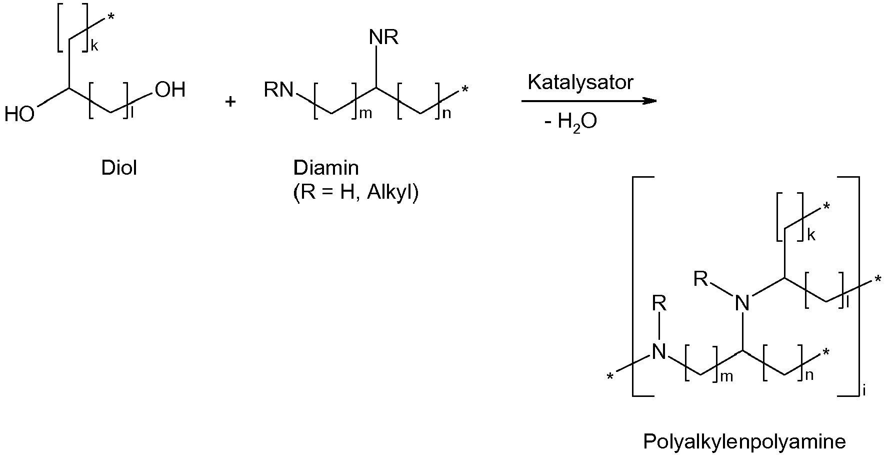

### Gleichung 1

Bevorzugte Alkylendiamine sind Ethylendiamin, 1,3-Propylendiamin, 1,2-Propylendiamin, Butylendiamin, beispielsweise 1,4-Butylendiamin oder 1,2-Butylendiamin, Diaminopentan, beispielsweise 1,5-Diaminopentan oder 1,2-Diaminopentan, Diaminohexan, beispielsweise 1,6-Diaminohexan oder 1,2-Diaminohexan, Diaminoheptan, beispielsweise 1,7-Diaminoheptan oder 1,2-Diaminoheptan, Diaminooctan, beispielsweise 1,8-Diaminooctan oder 1,2-Diaminooctan, Diaminononan, beispielsweise 1,9-Diaminononan oder 1,2-Diaminononan, Diaminodecan, beispielsweise 1,10-Diaminodecan oder 1,2-Diaminodecan, Diaminoundecan, beispielsweise 1,11-Diaminoundecan oder 1,2-Diaminoundecan, Diaminododecan, beispielsweise 1,12-Diaminododecan oder 1,2-Diaminododecan, 3,3'-Dimethyl-4,4'-diaminodicyclohexylmethan, 4,4'-Diaminodicyclohexylmethan, Isophorondiamin, 2,2-Dimethylpropan-1,3-diamin, 4,7,10-Trioxatridecan-1,13-diamin, 4,9-Dioxadodecan-1,12-diamin, Polyetheramine, Piperazin, 3-(Cyclohexyl-amino)propaylamin, 3-(Methylamino)propylamin, N,N-Bis(3-aminopropyl)methylamin.

Besonders bevorzugt sind 1,9-Nonandiol, 1,2-Decandiol, 1,10-Decandiol, 1,2-Dodecandiol, 1,12-Dodecandiol, Ethandiol, 1,2-Propandiol, 1,3-Propandiol.

Bevorzugte Alkylendiamine sind Ethylendiamin, 1,3-Propylendiamin, 1,2-Propylendiamin, Butylendiamin, beispielsweise 1,4-Butylendiamin oder 1,2-Butylendiamin, Diaminopentan, beispielsweise 1,5-Diaminopentan oder 1,2-Diaminopentan, 1,5-Diamino-2-methylpentan, Diaminohexan, beispielsweise 1,6-Diaminohexan oder 1,2-Diaminohexan, Diaminoheptan, beispielsweise 1,7-Diaminoheptan oder 1,2-Diaminoheptan, Diaminooctan, beispielsweise 1,8-Diaminooctan oder 1,2-Diaminooctan, Diaminononan, beispielsweise 1,9-Diaminononan oder 1,2-Diaminononan, Diaminodecan, beispielsweise 1,10-Diaminodecan oder 1,2-Diaminodecan, Diaminoundecan, beispielsweise 1,11-Diaminoundecan oder 1,2-Diaminoundecan, Diaminododecan, beispielsweise 1,12-Diaminododecan oder 1,2-Diaminododecan, 3,3'-Dimethyl-4,4'-diaminodicyclohexylmethan, 4,4'-Diaminodicyclohexylmethan, Isophorondiamin, 2,2-Dimethylpropan-1,3-diamin, 4,7,10-Trioxatridecan-1,13-diamin, 4,9-Dioxadodecan-1,12-diamin, Polyetheramine, Piperazin, 3-(Cyclohexyl-amino)propaylamin, 3-(Methylamino)propylamin, N,N-Bis(3-aminopropyl)methylamin.

Besonders bevorzugt sind Ethylendiamin, 1,2-Propylendiamin, 1,3-Propylendiamin, 1,5-Diamino-2-methylpentan, Hexamethylendiamin, 1,9-Diaminononan, 1,10-Diaminodecan, 1,12-Diaminododecan.

Besonders bevorzugt wird die Umsetzung von entweder (i) Ethylendiamin oder (ii) 1,3-Propylendiamin mit entweder a) 1,2-Decandiol oder b) 1,2-Dodecandiol oder (iii) 1,2-Propylendiamin mit entweder a) 1,2-Decandiol oder b) 1,2-Dodecandiol in Gegenwart eines Katalysators zum Polyalkanolpolyamin.

Die Zahl der sich wiederholenden-Einheiten i des Polyamins liegt im Allgemeinen zwischen 3 und 50 000.

Die so erhaltenen Polyalkanolamine können als Endgruppen an den Kettenenden sowohl NH₂- als auch OH-Gruppen tragen.

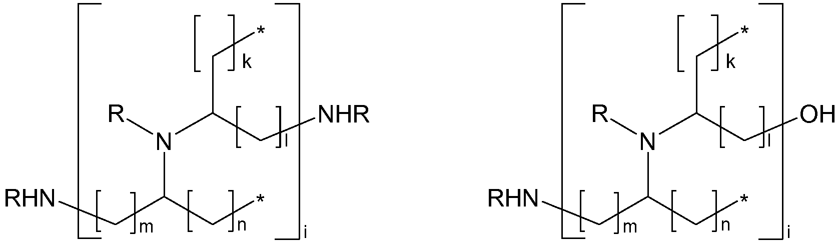

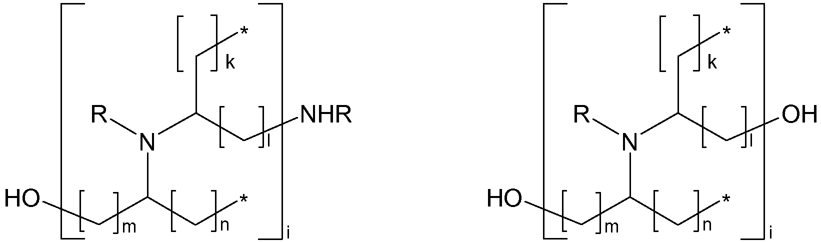

Mit bevorzugt
- R: unabhängig voneinander, gleich oder verschieden H, C₁-C₅₀-Alkyl,
- I, m: unabhängig voneinander, gleich oder verschieden ganze Zahl aus dem Bereich von 1 bis 50, bevorzugt von 1 bis 30, besonders bevorzugt von 1 bis 20,
- n, k: unabhängig voneinander, gleich oder verschieden ganze Zahl aus dem Bereich von 0 bis 50, bevorzugt von 0 bis 30, besonders bevorzugt von 0 bis 20,
- i: ganze Zahl aus dem Bereich von 3 bis 50000.

Das zahlenmittlere Molekulargewicht Mn der erhaltenen Polyalkanolamine beträgt im Allgemeinen von 200 bis 2 000 000, bevorzugt von 400 bis 750 000 und besonders bevorzugt von 400 bis 100 000. Der Wert des Polydispersitätsindex (Mw/Mn) liegt im Allgemeinen im Bereich von 1,2 bis 20, bevorzugt von 1,5-7,5. Die kationische Ladungsdichte (bei pH 4-5) liegt im Allgemeinen im Bereich von 0,1 bis 22 mequ/g Trockensubstanz, bevorzugt im Bereich von 0,5 bis10 mequ/g, besonders bevorzugt im Bereich von 0,5 bis 5 mequ/g.

Die nach dem erfindungsgemäßen Verfahren erhaltenen lipophilen Polyalkanolamine können sowohl in linearer Form als auch in verzweigter bzw. mehrfach-verzweigter Form vorliegen, als auch ringförmige Struktureinheiten aufweisen:

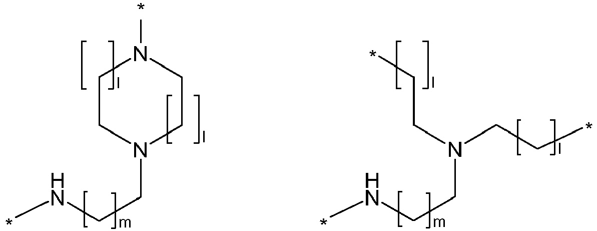

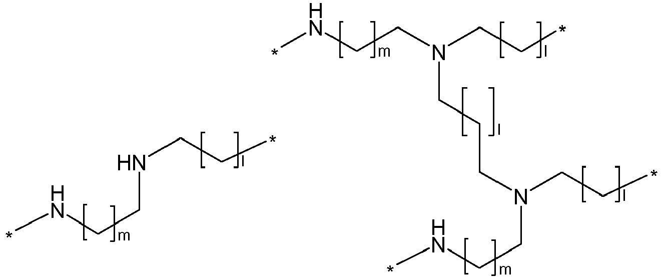

Dabei ist die Verteilung der Struktureinheiten (linear, verzweigt bzw. cyclisch) statistisch. Die so erhaltenen lipophilen Polyalkanolamine unterscheiden sich von den aus Ethylenimin hergestellten Polyethyleniminen insbesondere durch die vorliegenden OH-Endgruppen sowie durch die lipophilen Alkylen- bzw. Alkylgruppen.

In einer weiteren bevorzugten Ausführungsform der Erfindung wird ein linearer oder verzweigter Aminoalkohol mit fünf oder mehr C-Atomen in Gegenwart eines Katalysators umgesetzt:

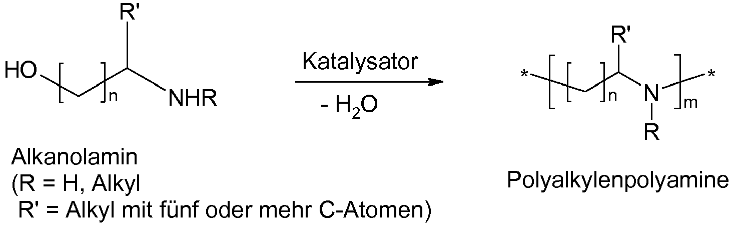

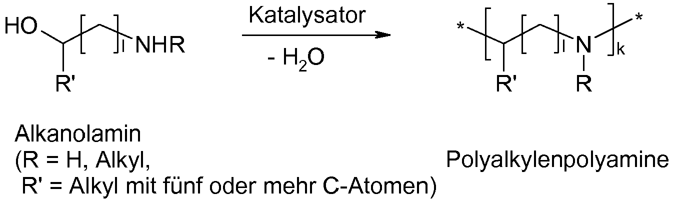

Beispiele sind 2-Aminohexan-1-ol, 5-Amino-2,2-dimethylpentanol, 2-Aminohexan-1-ol, 2-Aminoheptan-1-ol, 2-Aminooctan-1-ol, 2-Aminononan-1-ol, 2-Aminodecan-1-ol, 2-Aminoundecan-1-ol, 2-Aminodocedan-1-ol, 2-Aminotridecan-1-ol, 2-Aminoisohexan-1-ol, 2-Aminoisoheptan-1-ol, 2-Aminoisooctan-1-ol, 2-Aminoisononan-1-ol, 2-Aminoisodecan-1-ol, 2-Aminoisoundecan-1-ol, 2-Aminoisodocedan-1-ol, 2-Aminoisotridecan-1-ol.

In einer weiteren bevorzugten Ausführungsform der Erfindung wird ein linearer alpha, omega-Aminoalkohol mit fünf oder mehr C-Atomen in der Alkylenkette in Gegenwart eines Katalysators umgesetzt.

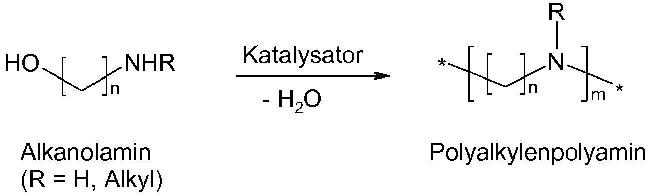

Beispiele sind Alkanolamine wie 6-Aminohexan-1-ol, 7-Aminoheptan-1-ol, 8-Aminooctan-1-ol, 9-Aminononan-1-ol, 10-Aminodecan-1-ol, 11-Aminoundecan-1-ol, 11-Aminoundecan-1-ol, 12-Aminododecan-1-ol, 13-Aminotridecan-1-ol.

In einer weiteren bevorzugten Ausführungsform der Erfindung wird ein linearer alpha, beta-Aminoalkohol mit fünf oder mehr C-Atomen in der Alkylgruppe in Gegenwart eines Katalysators umgesetzt.

Beispiele sind Alkanolamine wie 2-Aminoheptan-1-ol, 2-Aminooctan-1-ol, 2-Aminononan-1-ol, 2-Aminodecan-1-ol, 2-Aminoundecan-1-ol, 2-Aminoundecan-1-ol, 2-Aminododecan-1-ol, 2-Aminotridecan-1-ol.

Der Katalysator ist vorzugsweise ein Übergangsmetallkomplexkatalysator, der ein oder mehrere verschiedene Metalle der Nebengruppen des Periodensystems enthält, bevorzugt mindestens ein Element der Gruppen 8, 9 und 10 des Periodensystems, besonders bevorzugt Ruthenium oder Iridium. Der Katalysator liegt während der Umsetzung homogen gelöst im Reaktionsmedium vor.

Die genannten Nebengruppenmetalle liegen bevorzugt in Form von Komplexverbindungen vor. Es kommen zahlreiche verschiedene Liganden in Frage.

Geeignete, in den Übergangsmetallkomplexverbindungen vorliegende Liganden sind beispielsweise mit Alkyl oder Aryl substituierte Phosphine, mehrzähnige, mit Alkyl oder Aryl substituierte Phosphine, welche über Arylen- oder Alkylengruppen verbrückt sind, stickstoff-heterocyclische Carbene, Cyclopentadienyl und Pentamethylcyclopentadienyl, Aryl, Olefin-Liganden, Hydrid, Halogenid, Carboxylat, Alkoxylat, Carbonyl, Hydroxid, Trialkylamin, Dialkylamin, Monoalkylamin, Stickstoffaromaten wie Pyridin oder Pyrrolidin und mehrzähnige Amine. Der metallorganische Komplex kann einen oder mehrere verschiedene der genannten Liganden enthalten.

Bevorzugte Liganden sind (einzähnige) Phosphine oder (mehrzähnige) Polyphosphine, beispielsweise Diphosphine, mit mindestens einem unverzweigten oder verzweigten, acyclischen oder cyclischen, aliphatischen, aromatischen oder araliphatischen Rest mit 1 bis 20, bevorzugt 1 bis 12 C-Atomen. Beispiele für verzweigten cycloaliphatische und araliphatische Resten sind -CH₂-C₆H₁₁ und -CH₂-C₆H₅. Als geeignete Reste seinen beispielsweise genannt: Methyl, Ethyl, 1-Propyl, 2-Propyl, 1-Butyl, 2-Butyl, 1-(2-Methyl)propyl, 2-(2-Methyl)propyl, 1-Pentyl, 1-Hexyl, 1-Heptyl, 1-Octyl, 1-Nonyl, 1-Decyl, 1-Undecyl, 1-Dodecyl, Cyclopentenyl, Cyclohexyl, Cycloheptyl und Cyclooctyl, Methylcyclopentyl, Methylcyclohexyl, 1-(2-Methyl)-pentyl, 1-(2-Ethyl)-hexyl, 1-(2-Propylheptyl), Adamantyl und Norbonyl, Phenyl, Tolyl und Xylyl sowie 1-Phenylpyrrol, 1-(2-Methoxyphenyl)-pyrrol, 1-(2,4,6-Trimethyl-phenyl)- imidazol und 1-Phenylindol. Die Phosphin-Gruppe kann einen, zwei oder drei der genannten unverzweigten oder verzweigten acyclischen oder cyclischen, aliphatischen, aromatischen oder araliphatischen Reste enthalten. Diese können gleich oder verschieden sein.

In den genannten unverzweigten oder verzweigten, acyclischen oder cyclischen, aliphatischen, aromatischen oder araliphatischen Resten können einzelne Kohlenstoffatome auch durch weitere Phosphingruppen substituiert sein. Somit sind auch mehrzähnige, beispielsweise zwei- oder dreizähnige Phosphinliganden umfasst, deren Phosphingruppen durch Alkylen- oder Arylengruppen verbrückt sind. Vorzugsweise sind die Phosphingruppen durch 1,2-Phenylen-, Methylen-, 1,2-Ethylen-, 1,2-Dimethyl-1,2-Ethylen-, 1,3-Propylen-, 1,4-Butylen- und 1,5-Propylen-Brücken verbrückt.

Besonders geeignete einzähnige Phosphinliganden sind Triphenylphosphin, Tritolylphosphin, Tri-n-butylphosphin, Tri-n-octylphosphin, Trimethylphosphin und Triethylphosphin, Triphenylphosphantrisulfonat, Triethanolphosphin, 1,3,5-triaza-7-phosphatricyclo[3.3.1.13,7]decan, Di(1-adamantyl)-n-butylphosphin, Di(1-adamantyl)benzylphosphin, 2-(Dicyclohexylphosphino)-1-phenyl-1 H-pyrrol, 2-(Dicyclohexylphosphino)-1-(2,4,6-trimethyl-phenyl)-1H-imidazol, 2-(Dicyclohexylphosphino)-1-phenylindol, 2-(Di-tert-butylphosphino)-1-phenylindol, 2-(Dicyclohexylphosphino)-1-(2-methoxyphenyl)-1H-pyrrol, 2-(Di-tert-butylphosphino)-1-(2-methoxyphenyl)-1 H-pyrrol und 2-(Di-tert-butyl-phosphino)-1-phenyl-1H-pyrrol. Ganz besonders bevorzugt sind Tri-n-butylphosphin, Tri-n-octylphosphin, Triphenylphosphantrisulfonat, Triethanolphosphin, 1,3,5-triaza-7-phosphatricyclo[3.3.1.13,7]decan, Di(1-adamantyl)-n-butylphosphin, Di(1-adamantyl)benzylphosphin, 2-(Dicyclohexylphosphino)-1-phenyl-1 H-pyrrol, insbesondere wenn, wie bereits beschrieben, nach der Umsetzung ein polares Lösungsmittel zugesetzt wird. Hierbei sind diejenigen Liganden ganz bevorzugt, die mit Ru wasserlösliche Komplexe bilden.

Besonders geeignete mehrzähnige Phosphinliganden sind Bis(diphenylphosphino)methan, 1,2-Bis(diphenylphosphino)ethan, 1,2-Dimethyl-1,2-bis(diphenylphosphino)ethan, 1,2-Bis(dicyclohexylphosphino)ethan, 1,2-Bis(diethylphosphino)ethan, 1,3-Bis(diphenylphosphino)propan, 1,4-Bis(diphenylphosphino)butan, 2,3-Bis(diphenyl-phosphino)butan, 1,3-Bis(diphenylphosphino)propan, 1,1,1-Tris(diphenyl-phosphinomethyl)ethan, 1,1'-Bis(diphenylphosphanyl)ferrocen und 4,5-Bis(diphenylphosphino)-9,9-dimethylxanthen.

Weiterhin sind stickstoff-heterocylische Carbene als besonders geeignete Liganden genannt. Bevorzugt sind 1-Butyl-3-methylimidazolin-2-yliden, 1-Ethyl-3-methylimidazolin-2-yliden, 1-Methylimidazolin-2-yliden, 1,3-Bis(2,4,6-trimethylphenyl)imidazolin-2-yliden, 1-Butyl-3-methylimidazolin-2-yliden und Dipropylimidazolin-2-yliden, insbesondere wenn, wie bereits beschrieben, nach der Umsetzung ein polares Lösungsmittel zugesetzt wird. Besonders bevorzugt sind 1-Butyl-3-methylimidazolin-2-yliden, 1-Ethyl-3-methylimidazolin-2-yliden, 1-Methylimidazolin-2-yliden. Hierbei sind diejenigen Liganden ganz bevorzugt, die mit Ru wasserlösliche Komplexe bilden.
Als besonders geeignete Liganden seien auch Cyclopentadienyl sowie dessen mit Alkyl, Aryl und/oder Hydroxy ein- bis fünffach-substiuierte Derivate wie beispielsweise Methylcyclopentadienyl, Pentamethylcyclopentadienyl, Tetraphenylhydroxycyclopentadienyl und Pentaphenylcyclopentadienyl genannt. Weiterhin besonders geeignete Liganden sind Indenyl und dessen substituierte Derivate. Ebenfalls besonders geeignete Liganden sind Hydroxid, Chlorid, Hydrid und Carbonyl, insbesondere wenn, wie bereits beschrieben, nach der Umsetzung ein polares Lösungsmittel zugesetzt wird.

Der Übergangsmetallkomplexkatalysator kann mehrere verschiedene aller oben genannten Liganden enthalten. Hierbei sind diejenigen Liganden bzw. Ligandenkombinationen ganz bevorzugt, die mit Ru wasserlösliche Komplexe bilden.

Die homogenen Katalysatoren können sowohl direkt in ihrer aktiven Form eingesetzt werden als auch ausgehend von üblichen Standardkomplexen wie beispielsweise [Ru(p-cymene)Cl₂]₂, [Ru(benzol)Cl₂]ₙ, [Ru(CO)₂Cl₂]ₙ, [Ru(CO)₃Cl₂]₂, [Ru(COD)(allyl)], [RuCl₃*H₂O], [Ru(acetylacetonat)₃], [Ru(DMSO)₄Cl₂], [Ru(PPh₃)₃(CO)(H)Cl], [Ru(PPh₃)₃(CO)Cl₂], [Ru(PPh₃)₃(CO)(H)₂], [Ru(PPh₃)₃Cl₂], [Ru(cyclopentadienyl)(PPh₃)₂Cl], [Ru(cyclopentadienyl)(CO)₂Cl], [Ru(cyclopentadienyl)(CO)₂H], [Ru(cyclopentadienyl)(CO)₂]₂, [Ru(pentamethylcyclopentadienyl)(CO)₂Cl], [Ru(pentamethylcyclopentadienyl)(CO)₂H], [Ru(pentamethylcyclopentadienyl)(CO)₂]₂, [Ru(indenyl)(CO)₂Cl], [Ru(indenyl)(CO)₂H], [Ru(indenyl)(CO)₂]₂, Ruthenocen, [Ru(binap)Cl₂], [Ru(bipyridin)₂Cl₂*2H₂O], [Ru(COD)Cl₂]₂, [Ru(pentamethylcyclopentadienyl)(COD)Cl], [Ru₃(CO)₁₂], [Ru(tetraphenylhydroxy-cyclopentadienyl)(CO)₂H], [Ru(PMe₃)₄(H)₂], [Ru(PEt₃)₄(H)₂], [Ru(P*n*Pr₃)₄(H)₂], [Ru(P*n*Bu₃)₄(H)₂], [Ru(P*n*Octyl₃)₄(H)₂], [IrCl₃*H₂O], KIrCl₄, K₃IrCl₆, [Ir(COD)Cl]₂, [Ir(cycloocten)₂Cl]₂, [Ir(ethen)₂Cl]₂, [Ir(cyclopentadienyl)Cl₂]₂, [Ir(pentamethylcyclopentadienyl)Cl₂]₂, [Ir(cyclopentadienyl)(CO)₂], [Ir(pentamethylcyclopentadienyl)(CO)₂], [Ir(PPh₃)₂(CO)(H)], [Ir(PPh₃)₂(CO)(Cl)], [Ir(PPh₃)₃(Cl)] unter Zugabe der entsprechenden Liganden, bevorzugt der oben genannten ein- oder mehrzähnigen Phosphinliganden oder der oben genannten stickstoff-heterocyclischen Carbene, erst unter den Reaktionsbedingungen erzeugt werden.

Die Menge der Metallkomponente des Katalysators, bevorzugt Ruthenium oder Iridium, beträgt im Allgemeinen 0,1 bis 5000 Gewichts-ppm, jeweils bezogen auf das gesamte flüssige Reaktionsgemisch.

Das erfindungsgemäße Verfahren kann sowohl in einem Lösungsmittel als auch ohne Lösungsmittel ausgeführt werden. Selbstverständlich kann das erfindungsgemäße Verfahren auch in einem Lösungsmittelgemisch durchgeführt werden.

Wird das erfindungsgemäße Verfahren in einem Lösungsmittel durchgeführt, so wird die Menge an Lösungsmittel häufig so gewählt, dass sich die Edukte (i) und (ii) im Lösungsmittel gerade lösen. Im Allgemeinen beträgt das Gewichtsverhältnis der Menge an Lösungsmittel zu der Menge der Edukte (i) und (ii) von 100 : 1 bis 0,1 : 1, bevorzugt von 10 : 1 bis 0,1 : 1.

Wird die Reaktion ohne Lösungsmittel durchgeführt, so liegen nach der Umsetzung, insbesondere nach dem Abkühlen auf Umgebungstemperatur, und gegebenenfalls nach Zugabe eines Lösungsmittels oder Lösungsmittelgemisches, eine unpolare Phase und eine polare wässrige Phase vor. Der homogene Katalysator ist nach der Reaktion bevorzugt in der polaren Phase gelöst, während das Produkt in der unpolaren Phase vorliegt. Liegt der Katalysator in der polaren Phase vor, so kann er durch Phasentrennung von Produkt abgetrennt werden. Liegt der Katalysator teilweise oder vollständig in der unpolaren Phase vor, so kann dieser im Produkt verbleiben oder durch eine gegebenenfalls mehrfache Extraktion mit einem geeigneten Lösungsmittel aus diesem abgereichert werden. Als Extraktionsmittel wird bevorzugt ein stark polares Lösungsmittel eingesetzt, welches nach dem Einengen gegebenenfalls zusammen mit dem extrahierten Katalysator wieder für die Umsetzung eingesetzt werden kann. Geeignete Extraktionsmittel sind z.B. Wasser, Methanol, Ethanol, Dimethylsulfoxid, Dimethylformamid, ionische Flüssigkeiten wie z.B. 1-Ethyl-3-methylimidazoliumhydrogensulfat oder 1-Butyl-3-methylimidazoliummethansulfonat. Möglich ist auch die Entfernung des Katalysators mit einem geeigneten Absorbermaterial. Eine Abtrennung kann auch durch Zufügen von Wasser oder einer ionischen Flüssigkeit zu der Produktphase erfolgen, falls die Reaktion in einem mit Wasser bzw. der ionischen Flüssigkeit nicht mischbaren Lösungsmittel durchgeführt wird. Löst sich der Katalysator dabei bevorzugt in Wasser bzw. der ionischen Flüssigkeit, kann er mit dem Lösungsmittel von der organischen Produktphase abgetrennt und gegebenenfalls erneut eingesetzt werden. Dies kann durch Wahl geeigneter Liganden bewirkt werden.

Wird die Reaktion in einem Lösungsmittel durchgeführt, so kann dieses mit dem Produkt mischbar sein und nach der Reaktion durch Destillation abgetrennt werden. Geeignete Lösungsmittel sind z. B. Toluol, Benzol, Xylol, Alkane, z.B. Hexane, Heptane oder Octane, acyclische und cyclische Ether wie Diethylether oder Tetrahydrofuran sowie Alkohole mit mehr als drei C-Atomen, bei denen die OH-Gruppe an ein tertiäres Kohlenstoffatom gebunden ist, beispielsweise tert-Amylalkohol. Bevorzugt sind Benzol, Toluol, Xylolen, Alkane, acyclische und cyclischen Ethern oder Alkohole mit mehr als drei C-Atomen, bei denen die OH-Gruppe an ein tertiäres Kohlenstoffatom gebunden ist, besonders bevorzugt ist Toluol und tert-Amylalkohol. Bei der Destillation können auch nicht umgesetzte, insbesondere unpolare, Edukte abgetrennt werden.

Es können auch Lösungsmittel eingesetzt werden, welche eine Mischungslücke mit dem Produkt oder den Edukten aufweisen. Durch geeignete Wahl der Liganden löst sich der Katalysator bevorzugt in der polaren Phase. Geeignete Lösungsmittel sind in diesem Fall z.B. Wasser, Sulfoxide wie Dimethylsulfoxid, Formamide wie Dimethylformamid, ionische Flüssigkeiten wie z.B. 1-Ethyl-3-methylimidazoliumhydrogensulfat und 1-Butyl-3-methylimidazoliummethansulfonat, bevorzugt Wasser und ionische Flüssigkeiten.

Das Lösungsmittel kann auch unter den Reaktionsbedingungen mit den Edukten und dem Produkt und einer polaren Phase wie Wasser bzw. ionischen Flüssigkeiten mischbar sein und erst nach dem Abkühlen eine zweite flüssige Phase ausbilden, welche das Produkt enthält. Der Großteil des Katalysators ist in der polaren Phase gelöst. Diese Phase kann darüber hinaus noch einen Anteil der Edukte enthalten. Der Katalysator kann dann zusammen mit der polaren Phase abgetrennt und wieder verwendet werden. Der in dem Produkt enthaltene Teil des Katalysators kann anschließend durch Extraktion, geeignete Absorbermaterialien wie beispielsweise Polyacrylsäure und deren Salze, sulfonierte Polystyrole und deren Salze, Aktivkohlen, Montmorillonite, Bentonite sowie Zeolithe abgetrennt werden oder aber in dem Produkt belassen werden.

Bei der Ausführungsform der zweiphasigen Reaktionsführung eignen sich als unpolare Lösungsmittel besonders Toluol, Benzol, Xylole, Alkane, wie Hexane, Heptane und Oktane, in Kombination mit polaren oder hydrophilen Liganden am Übergangsmetallkatalysator wie stickstoff-heterocyclischen Carbenen, polaren Phosphanen oder kationischen oder anionischen Liganden, wodurch sich der Übergangsmetallkatalysator in der polaren Phase anreichert. Bei dieser Ausführungsform, in welcher das Lösungsmittel mit dem Produkt eine unpolare Phase bildet und der Katalysator sowie gegebenenfalls nicht umgesetzte Edukte mit dem Reaktionswasser und gegebenenfalls einem weiteren nach der Reaktion zugegebenen Lösungsmittel eine polare Phase bildet, kann der Großteil des Katalysators und gegebenenfalls nicht umgesetzte Edukte durch einfache Phasentrennung von der Produktphase abgetrennt und wiederverwendet werden.

Falls flüchtige Nebenprodukte oder nicht umgesetzte Edukte oder auch das zugesetzte Lösungsmittel unerwünscht sind, können diese problemlos von dem Produkt durch Destillation abgetrennt werden.

Bei einer weiteren Ausführungsform wird die Reaktion in einem polaren Lösungsmittel, in Wasser oder einer ionischen Flüssigkeit durchgeführt. Das Produkt kann durch Zufügen eines unpolaren Lösungsmittels abgetrennt werden, welches das Produkt löst, mit dem für die Reaktion verwendeten Lösungsmittel aber nicht mischbar ist. Beispiele für das unpolare Lösungsmittel sind Toluol, Benzol, Alkane, wie Hexane, Heptane oder Oktane, and acyclische oder cyclische Ether, wie Diethylether oder Tetrahydrofuran. Wenn sich der Katalysator bevorzugt in der polaren Phase löst, kann er mit dem Lösungsmittel von der unpolaren Produktphase abgetrennt und gegebenenfalls erneut eingesetzt werden.

Die Reaktion erfolgt in der Flüssigphase bei einer Temperatur von im Allgemeinen 20 bis 250 °C. Bevorzugt beträgt die Temperatur mindestens 100 °C und bevorzugt höchstens 200 °C, insbesondere bevorzugt beträgt die Temperatur 130 bis 180 °C. Die Reaktion kann bei einem Gesamtdruck von 0.1 bis 25 MPa absolut, der sowohl der Eigendruck des Lösungsmittels bei der Reaktionstemperatur als auch der Druck eines Gases wie Stickstoff, Argon oder Wasserstoff sein kann, durchgeführt werden. Die mittlere Reaktionszeit beträgt im Allgemeinen 15 Minuten bis 100 Stunden.

Es kann auch vorteilhaft sein, das bei der Reaktion gebildete Wasser kontinuierlich aus dem Reaktionsgemisch zu entfernen. Das Reaktionswasser kann direkt durch Destillation aus dem Reaktionsgemisch oder als Azeotrop unter Zusatz eines geeigneten Lösungsmittels (Schleppers) und unter Verwendung eines Wasserabscheiders abgetrennt, oder durch Zusatz von Wasser entziehenden Hilfsstoffen entfernt werden.

Der Zusatz von Basen kann einen positiven Effekt auf die Produktbildung haben. Als geeignete Basen seien hier Alkalihydroxide, Erdalkalihydroxide, Alkalialkoholate, Erdalkalialkoholate, Alkali-Carbonate und Erdalkalicarbonate genannt, von welchen 0,01 bis 100 Äquivalente in Bezug auf den verwendeten Metallkatalysator eingesetzt werden können.

In einer bevorzugten Ausführungsform des erfindungsgemäßen Verfahrens befinden sich die Heteroatome O oder N eines der Edukte (i) aliphatische Aminoalkohole, (ii) aliphatische Diamine oder Polyamine oder aliphatischen Diole oder Polyole in alpha- und beta-Position an der Kette der C-Atome und damit an benachbarten C-Atomen.

In einer bevorzugten Ausführungsform des erfindungsgemäßen Verfahrens befinden sich die Heteroatome O oder N eines der Edukte (i) aliphatische Aminoalkohole, (ii) aliphatische Diamine oder Polyamine oder aliphatischen Diole oder Polyole in alpha- und omega-Position an der Kette der C-Atome und damit an den entgegengesetzten Enden der Kette der C-Atome.

Ein weiterer Gegenstand der Erfindung ist ein Verfahren zur Herstellung von Polyalkylenpolyaminen, die Hydoxygruppen, sekundäre Amine oder tertiäre Amine enthalten. Bevorzugt befinden sich die Hydoxygruppen, sekundäre Amine oder tertiäre Amine an einem terminalen Kohlenstoffatom einer Alkylengruppe und stellen somit eine Endgruppe dar. Bevorzugt enthalten diese Polyalkylenpolyamine Hydroxygruppen.Insbesondere werden diese Polyalkylenpolyamine in einem Verfahren durch die Polymerisation von Monomeren in einem Schritt erhalten.

Bevorzugt beträgt das Verhältnis der Anzahl von Hydroxy-Endgruppen zu Amin-Endgruppen (primär, sekundär, tertiär) von 10 : 1 zu 1 : 10, bevorzugt von 5 : 1 zu 1 : 5, besonders bevorzugt von 2 : 1 zu 1 : 2.

In einer weiteren bevorzugten Ausführungsform weisen solche Polyalkylenpolyamine, die Hydoxygruppen, sekundäre Amine oder tertiäre Amine enthalten nur Hydroxy-Endgruppen oder nur Amin-Endgruppen (primär, sekundär, tertiär) auf.

Die vorliegende Erfindung stellt Verfahren zur Herstellung von lipophilen Polyalkylenpolyaminen zur Verfügung, bei denen keine unerwünschten Koppelprodukte, beispielsweise Ammoniak, entstehen.

Die Erfindung wird durch die Beispiele näher erläutert ohne dass die Beispiele den Gegenstand der Erfindung einschränken.

### Beispiele:

Das mittlere Molekulargewicht der Oligomere wurde per Gel-Permeations-Chromatographie nach der Methode der Größenausschlusschromatographie bestimmt. Als Elutionsmittel wurde Hexafluorisopropanol mit 0,05 % Trifluoressigsäure-Kaliumsalz verwendet. Die Messung wurde bei 40 °C mit einer Durchflußgeschwindigkeit von 1 ml/min auf einer Styrol-Divinylbenzol-Copolymer-Säule (8 mm * 30 cm) mit einem RI-Differentialrefraktometer bzw. UV-Photometer als Detektor durchgeführt. Die Kalibrierung erfolgte mit eng verteilten PMMA-Standards.

Zur Messung der Hazen-Farbzahl (nach APHA) wird die Probe 1:2500 mit einem Verdünnungsmittel, das im Bereich von 380 bis 720 nm nicht absorbiert, verdünnt. Die Hazen-Farbzahl wird dann in einem Bereich von 380 bis 720 nm in 10 nm-Schritten ermittelt.

### Beispiel 1

In einem 250 ml Autoklaven mit Blattrührer wurden unter Inertbedingungen zum Ausschluß von Sauerstoff 0,20 g (0,71 mmol) [Ru(COD)Cl2], 0,50 g (2,9 mmol) 1-Butyl-3-methylimidazolium-chlorid, 12,1 g (0,06 mol) 1,2-Dodecandiol, 20,0 g (0,27 mol) 1,3-Propylendiamin, 0,50 g (4,46 mmol) Kalium-tert-butylat und 34 ml Toluol eingewogen. Das Reaktionsgemisch wurde im verschlossenen Autoklaven bei 150°C unter dem Eigendruck des Lösungsmittels für 20 h gerührt. Nach vollendeter Reaktion und Abkühlen wurden zum Reaktionsgemisch 5 mL Wasser gegeben und geschüttelt, wobei man eine Lösung (50,0 g) des Produktes in Toluol sowie eine wässrige Lösung (12,66 g) des Katalysators erhielt. Die Phasen wurden voneinander abgetrennt und die Katalysatorphase wurde für Beispiel 2 wieder verwendet. Aus der lipophilen Produktphase wurde das nichtumgesetzte Edukt und flüchtige Bestandteile am Rotationsverdampfer bei 20 mbar und 120°C entfernt, wobei 14,13 g des reinen Produktes erhalten wurden. Das Gewichtsmittel (RI) des erhaltenen Oligomers lag bei 1470 g/mol mit einer Dispersität (Mw/Mn) von 3,9. Dies entspricht einer mittleren Kettenlänge n des Oligomers (CH₂CH(C₁₀H₂₁) NHCH₂CH₂NH)ₙ von 6. Die Farbzahl betrug 74.

### Beispiel 2

In einem 250 ml Autoklaven mit Blattrührer wurden unter Inertbedingungen die Katalysatorphase aus Beispiel 1 (12,66 g), 12,1 g (0,06 mol) 1,2-Dodecandiol, 20,0 g (0,27 mol) 1,3-Propylendiamin, und 34 ml Toluol eingewogen. Das Reaktionsgemisch wurde im verschlossenen Autoklaven bei 150°C unter dem Eigendruck des Lösungsmittels für 20 h gerührt. Nach vollendeter Reaktion und Abkühlen wurden zum Reaktionsgemisch 20 ml Wasser gegeben und geschüttelt, wobei man eine Lösung (49,67 g) des Produktes in Toluol sowie eine wässrige Lösung (27,38 g) des Katalysators erhielt. Die Phasen wurden abgetrennt. Aus der Produktphase wurde das nichtumgesetzte Edukt und flüchtige Bestandteile am Rotationsverdampfer bei 20 mbar und 120°C entfernt, wobei 11,53 g des reinen Produktes erhalten wurden. Das Gewichtsmittel (RI) des erhaltenen Oligomers lag bei 1020 g/mol mit einer Dispersität (Mw/Mn) von 3,6. Dies entspricht einer mittleren Kettenlänge n des Oligomers (CH₂CH(C₁₀H₂₁) NHCH₂CH₂NH)ₙ von 4.2. Die Farbzahl betrug 1.

### Beispiel 3

In einem 250 ml Autoklaven mit Blattrührer wurden unter Inertbedingungen 0,20 g (0,71 mmol) [Ru(COD)Cl2], 0,50 g (2,9 mmol) 1-Butyl-3-methylimidazoliumchlorid, 12,1 g (0,06 mol) 1,2-Dodecandiol, 20,0 g (0,27 mol) 1,3-Propylendiamin, 0,50 g (4,46 mmol) Kalium-tert-butylat und 34 ml Toluol eingewogen. Im verschlossenen Autoklaven wurde Wasserstoff auf 40 bar aufgepresst. Anschließend wurde das Reaktionsgemisch auf 150 °C erhitzt und für 20 h gerührt. Nach vollendeter Reaktion und Abkühlen wurden zum Reaktionsgemisch 20 mL Wasser gegeben und geschüttelt, wobei man eine Lösung des Produktes in Toluol sowie eine wässrige Lösung des Katalysators erhielt. Die Phasen wurden abgetrennt. Aus der Produktphase wurde das nichtumgesetzte Edukt und flüchtige Bestandteile am Rotationsverdampfer bei 20 mbar und 120°C entfernt, wobei 11.97 g des reinen Produktes erhalten wurden. Das Gewichtsmittel (RI) des erhaltenen Oligomers lag bei 1470 g/mol mit einer Dispersität (Mw/Mn) von 3.9. Dies entspricht einer mittleren Kettenlänge n des Oligomers (CH₂CH(C₁₀H₂₁) NHCH₂CH₂NH)ₙ von 6. Zur Messung der Farbzahl wurde das Produkt 2500-fach in Toluol verdünnt. Die Farbzahl betrug 21.

### Beispiel 4

In einem 250 ml Autoklaven mit Blattrührer wurden unter Inertbedingungen 0,20 g (0,71 mmol) [Ru(COD)Cl2], 0,50 g (2,9 mmol) 1-Butyl-3-methylimidazoliumchlorid, 0,50 g (4,46 mmol) Kalium-tert-butylat, 9,71 g des Austrags aus Beispiel 1 und 34 ml Toluol eingewogen. Das Reaktionsgemisch wurde im verschlossenen Autoklaven bei 140°C unter dem Eigendruck des Lösungsmittels für 20 h gerührt. Nach vollendeter Reaktion und Abkühlen wurden zum Reaktionsgemisch 20 ml Wasser gegeben und geschüttelt, wobei man eine Lösung des Produktes in Toluol sowie eine wässrige Lösung des Katalysators erhielt. Die Phasen wurden abgetrennt. Aus der Produktphase wurde das nichtumgesetzte Edukt und flüchtige Bestandteile am Rotationsverdampfer bei 20 mbar und 120°C entfernt, wobei 8,82 g des reinen Produktes erhalten wurden. Das Gewichtsmittel (RI) des erhaltenen Oligomers lag bei 1740 g/mol mit einer Dispersität (Mw/Mn) von 3.7. Dies entspricht einer mittleren Kettenlänge n des Oligomers (CH₂CH(C₁₀H₂₁) NHCH₂CH₂NH)ₙ von 7.3. Zur Messung der Farbzahl wurde das Produkt 2500-fach in Toluol verdünnt. Die Farbzahl betrug 200.

### Beispiel 5

In einem 250 m IAutoklaven mit Blattrührer wurden unter Inertbedingungen 0,20 g (0,71 mmol) [Ru(COD)Cl2], 0,50 g (2,9 mmol) 1-Butyl-3-methylimidazoliumchlorid, 12,1 g (0,06 mol) 1,2-Dodecandiol, 20,0 g (0,27 mol) 1,3-Propylendiamin, 0,50 g (4,46 mmol) Kalium-tert-butylat und 34 ml Toluol eingewogen. Das Reaktionsgemisch wurde im verschlossenen Autoklaven bei 130°C unter dem Eigendruck des Lösungsmittels für 30 h gerührt. Nach vollendeter Reaktion und Abkühlen wurden zum Reaktionsgemisch 20 ml Wasser gegeben und geschüttelt, wobei man eine Lösung des Produktes in Toluol sowie eine wässrige Lösung des Katalysators erhielt. Die Phasen wurden abgetrennt. Aus der Produktphase wurde das nichtumgesetzte Edukt und flüchtige Bestandteile am Rotationsverdampfer bei 20 mbar und 120°C entfernt, wobei 14,06 g des reinen Produktes erhalten wurden. Das Gewichtsmittel (RI) des erhaltenen Oligomers lag bei 1110 g/mol mit einer Dispersität (Mw/Mn) von 4.3. Dies entspricht einer mittleren Kettenlänge n des Oligomers (CH₂CH(C₁₀H₂₁) NHCH₂CH₂NH)ₙ von 4.6. Die Farbzahl betrug 56.

### Beispiel 6

In einem 250 ml Autoklav mit Blattrührer wurden unter Inertbedingungen 0,20 g (0,71 mmol) [Ru(COD)Cl2], 0,50 g (2,9 mmol) 1-Butyl-3-methylimidazoliumchlorid, 12,1 g (0,06 mol) 1,2-Dodecandiol, 20,0 g (0,27 mol) 1,3-Propylendiamin, 0,50 g (4,46 mmol) Kalium-tert-butylat und 34 ml Toluol eingewogen. Das Reaktionsgemisch wurde im verschlossenen Autoklaven bei 170°C unter dem Eigendruck des Lösungsmittels für 10 h gerührt. Nach vollendeter Reaktion und Abkühlen wurden zum Reaktionsgemisch 20 ml Wasser gegeben und geschüttelt, wobei man eine Lösung des Produktes in Toluol sowie eine wässrige Lösung des Katalysators erhielt. Die Phasen wurden abgetrennt. Aus der Produktphase wurde das nichtumgesetzte Edukt und flüchtige Bestandteile am Rotationsverdampfer bei 20 mbar und 120°C entfernt, wobei 14,18 g des reinen Produktes erhalten wurden. Das Gewichtsmittel (RI) des erhaltenen Oligomers lag bei 1220 g/mol mit einer Dispersität (Mw/Mn) von 37. Dies entspricht einer mittleren Kettenlänge n des Oligomers (CH₂CH(C₁₀H₂₁) NHCH₂CH₂NH)ₙ von 5.1. Die Farbzahl betrug 73.

### Beispiel 7

In einem 250 ml Autoklaven mit Blattrührer wurden unter Inertbedingungen 0,20 g (0,71 mmol) [Ru(COD)Cl2], 0,50 g (2,9 mmol) 1-Butyl-3-methylimidazoliumchlorid, 12,1 g (0,06 mol) 1,2-Dodecandiol, 20,0 g (0,27 mol) 1,3-Propylendiamin, 0,50 g (4,46 mmol) Kalium-tert-butylat und 17 ml Toluol eingewogen. Das Reaktionsgemisch wurde im verschlossenen Autoklaven bei 150°C unter dem Eigendruck des Lösungsmittels für 20 h gerührt. Nach vollendeter Reaktion und Abkühlen wurden zum Reaktionsgemisch 20 ml Wasser gegeben und geschüttelt, wobei man eine Lösung des Produktes in Toluol sowie eine wässrige Lösung des Katalysators erhielt. Die Phasen wurden abgetrennt. Aus der Produktphase wurde das nichtumgesetzte Edukt und flüchtige Bestandteile am Rotationsverdampfer bei 20 mbar und 120°C entfernt, wobei 13,72 g des reinen Produktes erhalten wurden. Das Gewichtsmittel (RI) des erhaltenen Oligomers lag bei 1280 g/mol mit einer Dispersität (Mw/Mn) von 3.8. Dies entspricht einer mittleren Kettenlänge n des Oligomers (CH₂CH(C₁₀H₂₁) NHCH₂CH₂NH)ₙ von 5.3. Die Farbzahl betrug 69.

### Beispiel 8

In einem 250 ml Autoklaven mit Blattrührer wurden unter Inertbedingungen 0,25 g (0,89 mmol) [Ru(COD)Cl2], 0,30 g (1,48 mmol) tri-n-Butylphosphin, 12,1 g (0,069 mol) 1,2-Decandiol, 25,0 g (0,42 mol) 1,2-Ethylendiamin, 0,10 g (0,89 mmol) Kalium-tert-butylat und 30 ml Toluol eingewogen. Das Reaktionsgemisch wurde im verschlossenen Autoklaven bei 150°C unter dem Eigendruck des Lösungsmittels für 30 h gerührt. Nach vollendeter Reaktion und Abkühlen wurde ein brauner Austrag (64,3 g) erhalten, aus dem das nichtumgesetzte Edukt und flüchtige Bestandteile am Rotationsverdampfer bei 12 mbar und 110°C entfernt wurden. Es wurden 14,6 g des Produktes erhalten. Das Gewichtsmittel (RI) des erhaltenen Oligomers lag bei 1380 g/mol mit einer Dispersität (Mw/Mn) von 2.4. Dies entspricht einer mittleren Kettenlänge n des Oligomers (CH₂CH(C₁₀H₂₁) NHCH₂CH₂NH)n von 7.

## Patentansprüche

1. Verfahren zur Herstellung von lipophilen Polyalkylenpolyaminen durch homogen katalysierte Alkohol-Aminierung, bei dem
(i) aliphatische Aminoalkohole miteinander oder
(ii) aliphatische Diamine oder Polyamine mit aliphatischen Diolen oder Polyolen
unter Wasserabspaltung in Gegenwart eines homogenen Katalysators umgesetzt werden, **dadurch gekennzeichnet, dass** mindestens eines der Edukte aliphatische Aminoalkohole, aliphatische Diamine oder Polyamine oder aliphatische Diole oder Polyole eine Alkyl- oder Alkylengruppe mit fünf oder mehr Kohlenstoffatomen enthält und
nach der Umsetzung eine Phasentrennung in mindestens eine unpolare und mindestens eine polare Phase vorliegt, wobei
die lipophilen Polyalkylenpolyamine in der unpolaren Phase angereichert vorliegen.

2. Verfahren nach Anspruch 1, **dadurch gekennzeichnet, dass** mindestens eines der Edukte aliphatische Aminoalkohole, aliphatische Diamine oder Polyamine oder aliphatischen Diole oder Polyole eine Alkyl- oder Alkylengruppe mit von 5 bis 50 Kohlenstoffatomen enthält.

3. Verfahren nach Anspruch 1 oder 2, **dadurch gekennzeichnet, dass** der Katalysator angereichert in der polaren Phase vorliegt.

4. Verfahren nach den Ansprüchen 1 bis 3, **dadurch gekennzeichnet, dass** nach der Umsetzung dem Reaktionsgemisch ein polares Lösungsmittel zugesetzt wird.

5. Verfahren nach den Ansprüchen 1 bis 4, **dadurch gekennzeichnet, dass** der Katalysator ein Übergangsmetallkomplexkatalysator ist.

6. Verfahren nach den Ansprüchen 1 bis 5, **dadurch gekennzeichnet, dass** der Katalysator einen stickstoff-heterocyclischen Carbenliganden enthält.

7. Verfahren nach den Ansprüchen 1 bis 5, **dadurch gekennzeichnet, dass** der Katalysator einen einzähnigen oder mehrzähnigen Phosphin-Liganden enthält.

8. Verfahren nach den Ansprüchen 1 bis 7, **dadurch gekennzeichnet, dass** der Katalysator einen Liganden, ausgewählt aus der Gruppe bestehend aus Cyclopentadienyl, substituiertem Cyclopentadienyl, Indenyl und substituiertem Indenyl, enthält.

9. Verfahren den Ansprüchen 1 bis 8, **dadurch gekennzeichnet, dass** der Katalysator einen Liganden, ausgewählt aus der Gruppe bestehend aus Hydroxid, Hydrid, Carbonyl und Chlorid, enthält, insbesondere wenn ein polares Lösungsmittel gemäß Anspruch 4 eingesetzt wird.

10. Verfahren nach den Ansprüchen 1 bis 9, **dadurch gekennzeichnet, dass** die Umsetzung in Gegenwart eines Lösungsmittels oder Lösungsmittelgemisches durchgeführt wird.

11. Verfahren nach den Ansprüchen 1 bis 10, **dadurch gekennzeichnet, dass** sich die Heteroatome O oder N eines der Edukte aliphatische Aminoalkohole, aliphatische Diamine oder Polyamine oder aliphatischen Diole oder Polyole in alpha- und beta-Position an der Kette der C-Atome und damit an benachbarten C-Atomen befinden.

12. Verfahren nach den Ansprüchen 1 bis 10, **dadurch gekennzeichnet, dass** sich die Heteroatome O oder N eines der Edukte aliphatische Aminoalkohole, aliphatische Diamine oder Polyamine oder aliphatischen Diole oder Polyole in alpha- und omega-Position an der Kette der C-Atome und damit an den entgegengesetzen Enden der Kette der C-Atome befinden.

## Claims

1. A process for the preparation of lipophilic polyalkylenepolyamines by homogeneously catalyzed alcohol amination, in which
(i) aliphatic amino alcohols are reacted with one another or
(ii) aliphatic diamines or polyamines are reacted with aliphatic diols or polyols
with the elimination of water in the presence of a homogeneous catalyst,
wherein at least one of the starting materials aliphatic amino alcohols, aliphatic diamines or polyamines or aliphatic diols or polyols comprises an alkyl or alkylene group having five or more carbon atoms and,
after the reaction, a phase separation into at least one nonpolar phase and at least one polar phase is present, where
the lipophilic polyalkylenepolyamines are present in enriched form in the nonpolar phase.

2. The process according to claim 1, wherein at least one of the starting materials aliphatic amino alcohols, aliphatic diamines or polyamines or aliphatic diols or polyols comprises an alkyl or alkylene group having from 5 to 50 carbon atoms.

3. The process according to claim 1 or 2, wherein the catalyst is present in enriched form in the polar phase.

4. The process according to claims 1 to 3, wherein, after the reaction, a polar solvent is added to the reaction mixture.

5. The process according to claims 1 to 4, wherein the catalyst is a transition metal complex catalyst.

6. The process according to claims 1 to 5, wherein the catalyst comprises a nitrogen-heterocyclic carbene ligand.

7. The process according to claims 1 to 5, wherein the catalyst comprises a monodentate or polydentate phosphine ligand.

8. The process according to claims 1 to 7, wherein the catalyst comprises a ligand selected from the group consisting of cyclopentadienyl, substituted cyclopentadienyl, indenyl and substituted indenyl.

9. The process according to claims 1 to 8, wherein the catalyst comprises a ligand selected from the group consisting of hydroxide, hydride, carbonyl and chloride, in particular if a polar solvent according to claim 4 is used.

10. The process according to claims 1 to 9, wherein the reaction is carried out in the presence of a solvent or solvent mixture.

11. The process according to claims 1 to 10, wherein the heteroatoms 0 or N of one of the starting materials aliphatic amino alcohols, aliphatic diamines or polyamines or aliphatic diols or polyols are located in alpha and beta position on the chain of carbon atoms and thus on adjacent carbon atoms.

12. The process according to claims 1 to 10, wherein the heteroatoms 0 or N of one of the starting materials aliphatic amino alcohols, aliphatic diamines or polyamines or aliphatic diols or polyols are located in alpha and omega position on the chain of carbon atoms and thus at opposite ends of the chain of carbon atoms.

## Revendications

1. Procédé de fabrication de polyalkylène-polyamines lipophiles par amination d'alcools sous catalyse homogène, selon lequel
(i) des aminoalcools aliphatiques sont mis en réaction les uns avec les autres ou
(ii) des diamines ou polyamines aliphatiques sont mises en réaction avec des diols ou polyols aliphatiques, avec clivage d'eau, en présence d'un catalyseur homogène,
**caractérisé en ce qu'**au moins un des réactifs aminoalcools aliphatiques, diamines ou polyamines aliphatiques ou diols ou polyols aliphatiques contient un groupe alkyle ou alkylène contenant cinq atomes de carbone ou plus, et
après la réaction, une séparation de phase en au moins une phase apolaire et au moins une phase polaire est présente,
les polyalkylène-polyamines lipophiles s'accumulant dans la phase apolaire.

2. Procédé selon la revendication 1, **caractérisé en ce qu'**au moins un des réactifs aminoalcools aliphatiques, diamines ou polyamines aliphatiques ou diols ou polyols aliphatiques contient un groupe alkyle ou alkylène contenant 5 à 50 atomes de carbone.

3. Procédé selon la revendication 1 ou 2, **caractérisé en ce que** le catalyseur s'accumule dans la phase polaire.

4. Procédé selon les revendications 1 à 3, **caractérisé en ce qu'**un solvant polaire est ajouté au mélange réactionnel après la réaction.

5. Procédé selon les revendications 1 à 4, **caractérisé en ce que** le catalyseur est un catalyseur de complexe de métal de transition.

6. Procédé selon les revendications 1 à 5, **caractérisé en ce que** le catalyseur contint un ligand carbène hétérocyclique azoté.

7. Procédé selon les revendications 1 à 5, **caractérisé en ce que** le catalyseur contient un ligand phosphine monodentate ou polydentate.

8. Procédé selon les revendications 1 à 7, **caractérisé en ce que** le catalyseur contient un ligand choisi dans le groupe constitué par le cyclopentadiényle, le cyclopentadiényle substitué, l'indényle et l'indényle substitué.

9. Procédé selon les revendications 1 à 8, **caractérisé en ce que** le catalyseur contient un ligand choisi dans le groupe constitué par hydroxyde, hydrure, carbonyle et chlorure, notamment lorsqu'un solvant polaire est utilisé selon la revendication 4.

10. Procédé selon les revendications 1 à 9, **caractérisé en ce que** la réaction est réalisée en présence d'un solvant ou d'un mélange de solvants.

11. Procédé selon les revendications 1 à 10, **caractérisé en ce que** les hétéroatomes 0 ou N d'un des réactifs aminoalcools aliphatiques, diamines ou polyamines aliphatiques ou diols ou polyols aliphatiques se trouvent en position alpha et bêta dans la chaîne des atomes C et par conséquent sur des atomes C voisins.

12. Procédé selon les revendications 1 à 10, **caractérisé en ce que** les hétéroatomes 0 ou N d'un des réactifs aminoalcools aliphatiques, diamines ou polyamines aliphatiques ou diols ou polyols aliphatiques se trouvent en position alpha et oméga dans la chaîne des atomes C et par conséquent à des extrémités opposées de la chaîne des atomes C.
